# FASCICULE DE BREVET EUROPEEN

(11) **EP 0 794 936 B1**
(45) Date de publication et mention de la délivrance du brevet: **17.03.1999**
(21) Numéro de dépôt: 95941159.6
(22) Date de dépôt: 29.11.1995
(51) Int. Cl.: C07C 209/36, C07C 211/52

(54) **PROCEDE DE PREPARATION DE FLUOROANILINES A PARTIR DE COMPOSES BENZENIQUES FLUORES NITRES**
VERFAHREN ZUR HERSTELLUNG VON FLUOROANILINEN AUS FLUORONITROBENZOLEN
METHOD FOR PREPARING FLUOROANILINES FROM NITROFLUOROBENZENE COMPOUNDS

(30) Priorité: 30.11.1994 FR 9414395; 30.12.1994 FR 9415956
(43) Date de publication de la demande: 17.09.1997
(60) Demande divisionnaire: 98113371.3
(73) Titulaire: RHODIA CHIMIE, 92408 Courbevoie Cédex (FR)
(72) Inventeur: CORDIER, Georges, F-69340 Francheville (FR); GUIDOT, Gilbert, F-69300 Caluire-et-Cuire (FR); MARION, Philippe, F-69100 Villeurbanne (FR); MERCIER, Claude, F-69005 Lyon (FR)
(74) Mandataire: Le Guen, Gérard
(86) Numéro de dépôt international: FR9501575
(87) Numéro de publication internationale: WO9616926

(56) Documents cités:
- EP-A- 0 001 825
- EP-A- 0 458 006
- EP-A- 0 562 435
- FR-A- 2 664 590
- US-A- 2 619 503
- US-A- 3 361 819
- DATABASE WPI Section Ch, Week 8231 Derwent Publications Ltd., London, GB; Class E14, AN 82-63760E & DD,A,154 214 ( VEB LEUNA-WERK W ULBRICH) , 3 Mars 1982
- CHEMICAL ABSTRACTS, vol. 120, no. 19, 9 Mai 1994 Columbus, Ohio, US; abstract no. 244212, J. MASON ET AL. 'Synthesis of 2,4-difluoroaniline and 1,3-difluorobenzene from 1,2,4-trichlorobenzene' page 956; & SYNTH. COMMUN., vol. 24, no. 4, 1994 pages 529-532,

## Description

La présente invention concerne un procédé de préparation de fluoroanilines, notamment de difluoroanilines, à partir de composés benzéniques fluorés nitrés et éventuellement substitués.

Les fluoroanilines sont des précurseurs de synthèse qui présentent un intérêt tout particulier dans le domaine des médicaments et de la protection végétale.

Cependant, leur obtention n'est pas directe mais découle de procédés de synthèse multi-étapes, ce qui rend ces produits très coûteux. Dans le but de réduire le prix de revient de ces composés, il convient de mettre en oeuvre des étapes dont le rendement est le plus élevé possible, en particulier la dernière étape.

Les différents procédés de synthèse proposés pour atteindre les fluoroanilines passent par des composés fluorés nitrés qui sont ensuite réduits en amines. Une voie d'accès particulière consiste à préparer un nitrobenzène fluoré et en général chloré pour le soumettre ensuite à une opération d'hydrogénation pour réduire la fonction nitro en fonction amine tout en hydrogénolysant les atomes de chlore éventuels, qui sont ainsi remplacés par des atomes d'hydrogène.

Ainsi le brevet américain US-4 140 719 décrit une synthèse de 2,4-difluoroaniline mettant en oeuvre l'hydrogénation de 5-chloro 2,4-difluoro nitrobenzène avec un rendement de 84%.

De même, le brevet américain US-5 294 742 décrit l'hydrogénation de 2,6-dichloro 3,5-difluoro nitrobenzène pour donner de la 3,5-difluoroaniline avec un rendement de 88,9%.

De tels rendements sont acceptables au laboratoire mais impliquent un certain manque à gagner, qu'il conviendrait d'éviter en vue d'une application industrielle.

En outre, il est à noter que ces rendements s'accompagnent de taux de conversion quantitatifs, le reste du matériau de départ étant transformé en composés de type azo ou azoxy, mais aussi, comme l'ont constaté les présents inventeurs, en polyanilines par polycondensation, ces sous-produits étant réputés hautement toxiques. Leur présence, même à l'état de traces après purification est donc un inconvénient majeur en vue de l'utilisation des fluoroanilines dans la synthèse de médicaments ou de produits en contact avec l'environnement (notamment dans des produits pour l'agriculture).

Ainsi, la présente invention a pour but de proposer un procédé de préparation de fluoroanilines, notamment de difluoroanilines, à partir de composés benzéniques fluorés nitrés avec un rendement quasiment quantitatif, sans formation notable de sous-produits toxiques.

L'invention a en particulier pour but de proposer un procédé d'hydrogénation de tels composés benzéniques fluorés nitrés permettant d'effectuer la réduction desdits composés, et éventuellement l'hydrogénolyse de substituants halogènes autres que des atomes de fluor, de façon sélective vis-à-vis de l'hydrogénodéfluoration.

A cet effet, l'invention a pour objet un procédé pour la préparation d'un composé de formule (I) 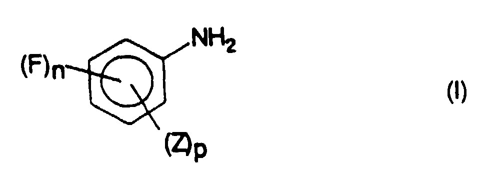 où
n vaut de 1 à 5,
Z est un radical stable dans des conditions d'hydrogénation catalytique,
p est inférieur à 5-n,
selon lequel on soumet un composé de formule (II) 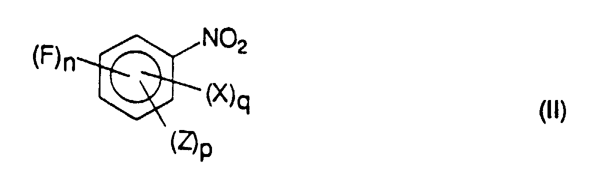 où
n, Z et p ont les significations précédentes,
X est un radical hydrogénolysable,
q vaut de 0 à 5-(n + p),
à une hydrogénation catalytique un milieu liquide contenant un catalyseur, sous pression d'hydrogène, réalisant une réaction de réduction catalytique et, le cas échéant, une réaction d'hydrogénolyse,
caractérisé en ce que le composé de formule (Il) est introduit progressivement dans ledit milieu de sorte que la teneur en composé de formule (Il) dans le liquide reste inférieure ou égale à 1000 ppm en masse, pour former sélectivement le composé de formule (I).

Comme composés de formule (Il) de départ, on vise particulièrement les composés où X représente un atome d'halogène, notamment de chlore, et notamment les chloro difluoro nitrobenzènes.

Le radical Z peut être tout radical hydrocarboné, comprenant éventuellement au moins un hétéroatome, non hydrogénolysable, c'est-à-dire qui n'est pas remplacé par un atome d'hydrogène dans des conditions d'hydrogénation catalytique. Un tel groupe peut être en particulier un groupe alkyle.

Selon l'invention, pour obtenir une bonne conversion et une bonne sélectivité, donc un bon rendement en le produit désiré, le composé de formule (II) est introduit progressivement dans le milieu d'hydrogénation de sorte que la teneur en composé de formule (II) dans le liquide réactionnel reste inférieure ou égale à 1000 ppm, de préférence inférieure ou égale à 500 ppm, notamment dans la gamme de 200 à 500 ppm.

L'introduction du composé benzénique de façon progressive, en discontinu ou en continu, dans le milieu d'hydrogénation permet d'éviter de façon remarquable la formation de sous-produits indésirables.

De préférence, on introduit le composé benzénique nitré en continu dans le milieu d'hydrogénation.

Cette introduction doit être réalisée de sorte que la teneur en composé de formule (Il) dans le liquide reste inférieure à 1000 ppm. De préférence, cette teneur est maintenue inférieure à 500 ppm, en particulier dans la gamme de 200 à 500 ppm. On peut cependant aussi bien choisir de maintenir cette teneur à une valeur très faible, notamment largement inférieure à 200 ppm. Dans un mode de réalisation particulier, la réaction d'hydrogénation peut être avantageusement conduite dans une enceinte munie de moyens de mesure de la teneur en composé de formule (Il) dans le liquide réactionnel, auxquels sont éventuellement asservis les moyens d'introduction du composé de formule (Il).

Avantageusement, le composé de formule (Il) est introduit progressivement dans un milieu d'hydrogénation liquide sous agitation, de façon à éviter des surconcentrations c'est-à-dire des concentrations locales supérieures à la concentration maximale souhaitée.

De préférence, le milieu liquide dans lequel est réalisée l'hydrogénation du composé de formule (II) est un liquide solvant à la fois pour le composé de formule (II) et pour les divers produits de la réaction.

En effet, le procédé selon l'invention est particulièrement efficace lorsque la réaction a lieu en solution dans un milieu liquide homogène, c'est-à-dire en présence d'une seule phase liquide. Ce milieu liquide homogène ou monophasique peut toutefois comprendre des particules solides en suspension introduites en tant que telles dans le milieu, à savoir des particules de catalyseur.

Cependant, la réaction peut également être conduite en milieu hétérogène, c'est-à-dire si les partenaires réactionnels sont insolubles ou partiellement solubles dans le milieu ; les conditions réactionnelles appropriées seront détaillées par la suite.

Le solvant est de préférence un solvant polaire, pouvant être protique ou aprotique. La constante diélectrique relative ∈ dudit solvant est avantageusement au moins égale à 5, de préférence inférieure ou égale à 50. Avantageusement, le solvant présente un indice accepteur A d'au moins 8, de préférence supérieur ou égal à 9. Pour la définition de l'indice accepteur A, on se reportera à l'ouvrage de C. Reichardt, Solvents and solvent effects in Organic Chemistry, 2ème édition, VCH (RFA), 1990, pp. 23-24. Sont également avantageux les solvants dont l'indice donneur D exprimé par la variation d'enthalpie (ΔH en kcal/mol) de l'association dudit solvant avec le pentachlorure d'antimoine est de 10 à 30. On peut utiliser notamment le méthanol ou l'acétate d'éthyle. Avantageusement, le solvant peut être choisi parmi les solvants miscibles à l'eau.

Lorsqu'on travaille en milieu alcoolique, il est préférable, pour éviter des réactions parasites dues au solvant, de limiter le pH du milieu à une valeur inférieure ou égale à 13.

Des solvants contenant du soufre susceptible d'être réduit dans les conditions de l'hydrogénation, par exemple le DMSO, sont à éviter car ils conduisent à des sous-produits non désirés qui peuvent en particulier rendre le catalyseur inactif. Un solvant soufré tel que le sulfolane est cependant acceptable car il est très difficile a réduire.

De manière générale, il peut être avantageux de travailler dans un solvant de point d'ébullition suffisamment éloigné de celui du produit pour faciliter l'isolement du produit de réaction par distillation.

Le catalyseur peut être choisi parmi tous les catalyseurs d'hydrogénation connus, notamment les métaux tels que le nickel, le palladium ou les métaux de la mine du platine, éventuellement sur un support minéral ou organique tel que le noir de carbone et/ou le charbon actif. Le palladium savère être très approprié lorsqu'on travaille en milieu hétérogène et la réaction est possible en travaillant à une température d'au moins environ 100°C et jusqu'à environ 150°C. Les zéros de position ne sont pas considérés comme des chiffres significatifs dans la présente description à moins qu'il en soit précisé autrement.

Le procédé de l'invention peut s'appliquer à la préparation de fluoroanilines par hydrogénation de fluoronitrobenzènes, c'est-à-dire des composés de formule (II) où q vaut 0.

Dans ce cas, le choix du catalyseur est moins crucial et on peut choisir notamment le charbon palladié.

Lorsque le composé de formule (Il) ne porte pas de substituant hydrogénolysable, c'est-à-dire si q = 0, l'hydrogénation catalytique ne réalise que la réaction de réduction de NO₂ en NH₂. Les conditions de température réactionnelle peuvent être de 0 à 150° C, de préférence de 30 à 130° C.

Le procédé de l'invention peut également s'appliquer à la préparation de fluoroanilines par hydrogénation catalytique de chloro fluoro nitrobenzènes, c'est-à-dire de composés de formule (Il) où p est nul et X représente CI, tout particulièrement à la préparation de difluoroanilines par hydrogénation catalytique de composés du type indiqué où q vaut 1 (chloro difluoro nitrobenzènes), où q vaut 2 (dichloro difluoro nitrobenzènes) ou bien 3 (trichloro difluoro nitrobenzènes). Dans ce cas particulier, le procédé de l'invention réalise alors la réduction du groupe nitro en amino et l'hydrogénolyse du ou des atomes de chlore.

La réduction du groupe nitro en groupe amino, qui conduit de façon transitoire ou effective au composé benzénique aminé fluoré et halogéné correspondant, et l'hydrogénolyse du ou des groupes X peuvent être concomitantes ou de préférence successives. L'hydrogénolyse peut avoir lieu sur le composé aminé en tant que composé intermédiaire ou intermédiaire de transition, ou bien concomitamment à la réduction du composé nitré.

De préférence, lorsque q est non nul et X est un atome d'halogène autre que le fluor, notamment un atome de chlore, les conditions d'hydrogénation sont adaptées pour former dans une étape a) un composé de formule (III) 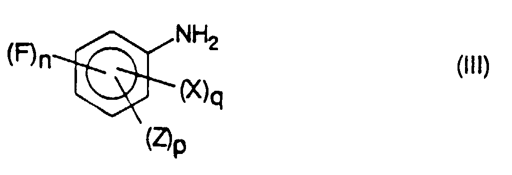 à partir du composé de formule (Il) par réduction catalytique, puis
dans une étape b) former le composé de formule (I) à partir du composé de formule (III) par hydrogénolyse.

Les conditions de l'hydrogénation peuvent être telles que le même catalyseur réalise les réactions de l'étape (a) et de l'étape (b). Un tel catalyseur peut comprendre du charbon palladié.

Dans ce cas, des conditions d'hydrogénation catalytique réalisant les réactions des étapes (a) et (b) de façon concomittante comprennent une température de 50 à 130°C. La pression d'hydrogène est avantageusement de 10⁵ à 5.10⁶ Pa dans ce cas.

Des conditions d'hydrogénation réalisant d'abord la conversion de sensiblement tout le composé de formule (II) en composé de formule (III) selon l'étape (a) catalysée par le charbon palladié comprennent une température de 0 à 70° C, en particulier inférieure à 60° C, de préférence inférieure ou égale à 50° C. La pression partielle d'hydrogène est de préférence de 10⁵ à 2.10⁶ Pa. Au-delà de 70° C, le charbon palladié catalyse efficacement la réaction de l'étape (b).

L'étape (b) peut être mise en oeuvre ensuite avec une température supérieure à 80° C, en particulier supérieure à 100° C, notamment à 120° C. La pression partielle d'hydrogène est avantageusement de 1,5.10⁶ à 5.10⁶ Pa au cours de cette étape.

Les conditions d'hydrogénation peuvent également être telles que l'on utilise un catalyseur pour l'étape (a), et un autre catalyseur, distinct du précédent, pour l'étape (b).

On peut choisir le catalyseur d'hydrogénation pour obtenir de façon sélective dans un premier temps le composé de formule (III), que l'on peut isoler. A cet égard, on préfère le nickel, notamment le nickel de Raney, ou un catalyseur métallique empoisonné d'une façon connue de la technique d'hydrogénation, par exemple du nickel de Raney empoisonné. On peut citer les catalyseurs métalliques empoisonnés au soufre tels que décrits dans FR-A-2 664 590 et FR-A-2 649 979, et les catalyseurs métalliques empoisonnés par un iodure tel que décrit dans FR-A-2 649 978. On préfère les catalyseurs légèrement empoisonnés, c'est-à-dire où la quantité de poison est insuffisante pour rendre le catalyseur totalement inactif. La réaction est alors conduite de préférence à une température inférieure à 80° C, de préférence à 70° C. La pression partielle d'hydrogène est de préférence de 10⁵ à 2.10⁶ Pa.

La réaction de l'étape (b) peut alors avoir lieu dans un second temps en utilisant un autre catalyseur ne faisant pas partie du groupe cité ci-dessus, qui est actif pour catalyser ladite hydrogénolyse. On peut utiliser en particulier notamment du charbon palladié. La réaction est alors conduite de préférence à une température supérieure à 80° C, en particulier supérieure à 100° C, notamment à 120° C. La pression partielle d'hydrogène est de préférence de 1,5.10⁶ à 5.10⁶ Pa dans cette étape.

Dans le cas où l'on désire séparer le composé de formule (III), c'est à dire simplement réduit par la réaction de l'étape (a), il est particulièrement avantageux d'opérer dans un solvant de point d'ébullition relativement élevé, pour pouvoir séparer soigneusement ce produit intermédiaire par distillation, éventuellement même au fur et à mesure de sa synthèse avant la fin de l'étape (a).

Comme gaz d'hydrogénation, on peut utiliser de l'hydrogène pur ou bien un mélange d'hydrogène et d'un gaz inerte, notamment d'azote.

Dans les conditions de température et de pression indiquées précédemment, la réaction de réduction du groupe nitro en amino (étape (a)) est complète si la quantité d'hydrogène n'est pas limitée.

La réaction d'hydrogénolyse des groupes X peut être limitée par l'utilisation d'un catalyseur sélectif, tel que le nickel de Raney partiellement empoisonné, ou par l'emploi de conditions de température et/ou de pression insuffisantes.

Lorsque X représente un atome d'halogène, la réaction de l'étape (b) libère le ou les atomes d'halogène sous forme d'acide halohydrique.

Il est alors avantageux d'opérer en présence d'une base pour neutraliser l'acide libéré. De manière générale, le pH devrait de préférence être maintenu à une valeur telle que l'essentiel de l'aniline formée, c'est-à-dire au moins 80 %, avantageusement au moins 90%, reste sous forme libre dans le milieu réactionnel.

La base ajoutée est de préférence une base alcaline, notamment de la soude, qui peut être introduite dans le milieu sous forme de solution aqueuse.

On peut également utiliser des oxydes alcalino-terreux, tels que notamment MgO et CaO. Ces composés sont cependant difficilement solubles dans l'eau, ou du moins ont une cinétique de dissolution très lente, de sorte que leur utilisation peut introduire beaucoup de solides dans le milieu d'hydrogénation et ainsi ne pas permettre d'atteindre des résultats aussi bons que ceux obtenus dans les conditions adaptées à une solution homogène (abstraction faite du catalyseur solide). Comme on l'a dit précédemment, on peut toutefois travailler sans problème en milieu hétérogène dans des conditions appropriées.

En variante, on peut également utiliser comme agent absorbeur d'acidité une résine échangeuse d'ions basique.

Lorsqu'on travaille en milieu alcoolique, pour éviter des réactions parasites de substitution du noyau aromatique par des espèces provenant du solvant, il est préférable que la base utilisée soit relativement faible, notamment que son pKa soit au moins égal à celui de l'ion Mg²⁺ et inférieur à 13 ; ou bien de réguler le pH en ajoutant progressivement la base de manière à éviter que le pH ne dépasse 13. On peut également réguler le pH en utilisant un tampon.

Dans le cas où on utilise une solution aqueuse basique, il est préférable de choisir pour constituer le milieu d'hydrogénation, un solvant miscible à l'eau de façon à ce que l'hydrogénation se passe en solution homogène.

Au cours de la réaction, il est préférable que la quantité de réactifs en présence soit telle que la concentration des produits (tels que la base azotée, ou son halohydrate, ou les sels issus de la réaction de l'acide halohydrique libéré avec la base introduite) reste inférieure à la limite de solubilité de chacun de ces produits dans le milieu réactionnel. De préférence, on évite la précipitation de matières solides dans le milieu réactionnel en maintenant les concentrations des espèces en solution à une valeur inférieure à 90%, plus particulièrement à une valeur inférieure à 80% de leur limite de solubilité.

A cet égard, la modification de pH impliquée par l'ajout de base au milieu réactionnel peut être avantageusement mise à profit pour modifier lesdites limites de solubilité et continuer d'opérer en solution homogène.

Toutefois, il n'est pas obligatoire de travailler en milieu homogène. La réaction est possible en milieu hétérogène en travaillant en particulier à l'étape b) à une température d'au moins environ 100°C et jusqu'à environ 150°C (les zéros de position n'étant pas considérés comme des chiffres significatifs). De préférence, on utilisera un catalyseur au palladium.

On peut utiliser toutes les bases connues en quantité variable mais, lorsque ces bases se présentent sous forme de sels peu solubles, comme la soude ou la potasse, il est préférable de se trouver à une concentration inférieure ou égale à 1 mol/l,en particulier inférieure ou égale à 0,5 mol/l.

La base est avantageusement introduite dans le milieu réactionnel au fur et à mesure de la libération d'acide halohydrique au cours de la réaction.

En variante, on peut n'opérer en présence de base qu'à partir de la fin de l'introduction progressive de composé de formule (Il), en élevant éventuellement la température du milieu réactionnel. Dans ce cas, on peut également introduire la base au fur et à mesure de la libération d'acide halohydrique.

Lorsqu'on utilise un catalyseur sélectif dans le but de ne réaliser dans un premier temps que la réaction de l'étape (a), il est préférable de ne pas opérer en présence d'une base pour limiter les risques de réactions secondaires.

A l'issue de l'introduction progressive du composé de formule (Il), ce dernier a été totalement réduit en amine et le milieu réactionnel peut comprendre uniquement le composé de formule (III) ou bien un mélange de composé de formule (III) et de composé de formule (Il) selon les conditions réactionnelles.

Si le mélange réactionnel en fin d'addition contient du composé de formule (III), on peut poursuivre l'opération d'hydrogénation pour atteindre le composé de formule (I) avec un rendement quantitatif.

A ce stade, on peut poursuivre l'hydrogénation catalytique directement sur le mélange réactionnel. Si l'on a opéré jusqu'à présent avec un catalyseur sélectif ne permettant pas la réaction (b), il convient d'introduire dans le milieu d'hydrogénation un autre catalyseur actif cette fois pour cette réaction.

En variante, on peut également séparer le produit réactionnel de l'hydrogénation, sous forme des composés de formule (I) et de formule (III), ou sous forme de leur mélange.

Le composé de formule (III) peut alors être soumis, seul ou en mélange avec le composé de formule (I), à une nouvelle opération d'hydrogénation catalytique dans une enceinte d'hydrogénation contenant un milieu liquide solvant pour les anilines fluorées en présence d'un catalyseur d'hydrogénation et d'une base. Le milieu réactionnel peut être identique ou différent du milieu réactionnel de la première hydrogénation, en respectant de préférence les variantes avantageuses énoncées précédemment (notamment en choisissant un solvant polaire, avantageusement miscible à l'eau...).

Toutes les variantes énoncées ci-dessus permettent d'atteindre une conversion quantitative du composé benzénique de départ avec un rendement en aniline fluorée quasiment quantitatif.

De préférence, l'hydrogénation est conduite dans un seul milieu en enchaînant les réactions (a) et (b) pour obtenir in fine le composé de formule (I). On opère de préférence en présence de charbon palladié en tant que catalyseur. Le catalyseur peut comporter avantageusement de 5 à 15 % de palladium. De préférence, la température est maintenue inférieure à 70° C pendant l'introduction progressive du composé de formule (Il), puis elle est portée à une valeur supérieure à 80° C, de préférence supérieure à 100-120° C pour achever la réaction.

L'aniline fluorée obtenue peut être séparée du milieu réactionnel en fin d'hydrogénation, notamment par distillation.

A la fin de la réaction d'hydrogénation, on peut atteindre une concentration en amine pouvant aller jusqu'à 50% en poids du milieu réactionnel. On atteint très facilement des concentrations supérieures à 20% en poids.

Cette possibilité d'obtenir des produits finals très concentrés dans le milieu réactionnel permet d'améliorer la productivité des outils, ce qui représente un avantage supplémentaire du procédé de l'invention, s'ajoutant à son rendement élevé.

Le procédé selon la présente invention peut s'appliquer fort avantageusement à la préparation de 2,4-difluoroaniline de formule (la) 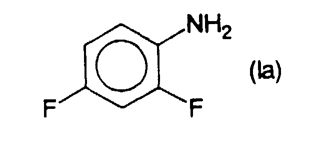 par hydrogénation catalytique de composés de formule (lla) 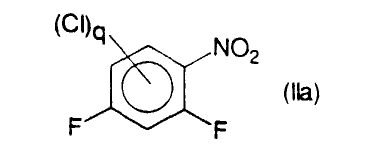 dans laquelle q est un entier de 0 à 2, le ou les atomes de chlore éventuels étant en position méta par rapport au groupe nitro.

L'hydrogénation de ces composés conduisant au même produit final, la réaction peut être réalisée avec un mélange de ces composés.

Le schéma 1 propose une synthèse multi-étape de la 2,4-difluoroaniline utilisant cette hydrogénation et partant de précurseurs très simples.

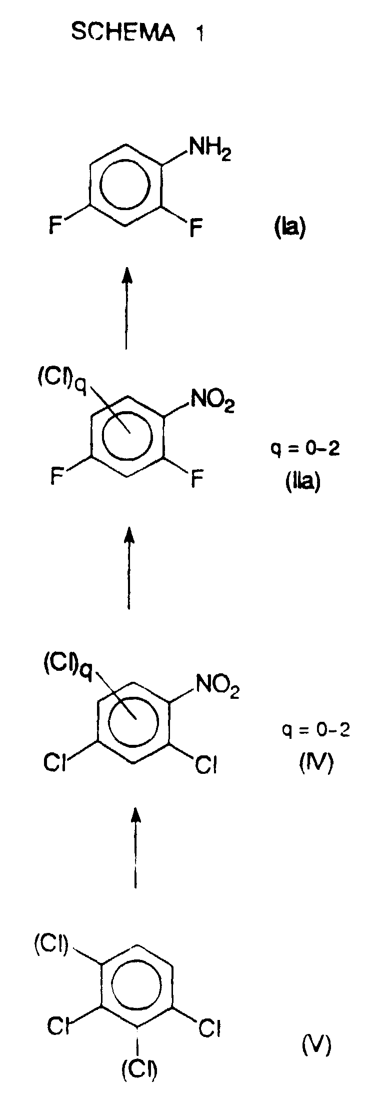

Un composé ou un mélange de composés de formule (lla) 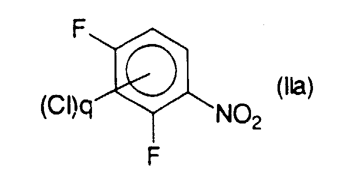 où q vaut de 0 à 2, choisis parmi le 2,4-difluoronitrobenzène et ses dérivés chlorés en position 3 ou 5, ou en positions 3 et 5, peut être obtenu par réaction d'un composé ou d'un mélange de composés de formule (IV) 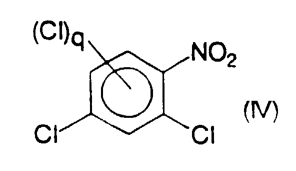 choisis respectivement parmi le 2,4-dichloronitrobenzène et ses dérivés chlorés en position 3 ou 5, ou en positions 3 et 5, avec une source de fluorures.

Ce sont les atomes de chlore en position ortho et para qui sont substitués par des atomes de fluor. Plus le noyau est appauvri, notamment par les autres substituants CI, plus l'échange est facile.

Comme source de fluorure, on utilise classiquement un fluorure alcalin, notamment un fluorure de potassium ou de césium, ou un fluorure d'ammonium quaternaire, en léger excès calculé par rapport aux deux atomes de chlore en ortho et para à substituer par du fluor.

La réaction a lieu de préférence dans un solvant polaire aprotique sec connu de l'homme du métier, présentant notamment les caractéristiques de constante diélectrique ∈ et d'indice donneur D indiquées précédemment.

Avantageusement, le composé ou le mélange de composés de formule (IV) est obtenu par réaction d'un composé ou d'un mélange de composés de formule (V) 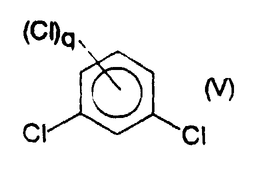 choisis respectivement parmi le 1,3-dichlorobenzène et ses dérivés chlorés en position 2 ou 4, ou en positions 2 et 4, avec un réactif de nitration de composés aromatiques, notamment avec de l'acide nitrique.

La fonction nitro s'introduit en position 6 à 96% sur le 1,2,3-trichloro benzène, et en position 5 à 90% sur le 1,2,4-trichlorobenzène.

Quel que soit le composé benzénique chloré de formule (V) utilisé au début de la séquence réactionnelle, le produit final est la 2,4-difluoroaniline de formule (la). On peut donc travailler sans aucun danger avec des mélanges de départ contenant divers composés benzéniques chlorés.

De même, le procédé selon la présente invention peut s'appliquer à la préparation de 3,5-difluoroaniline de formule (Ib) 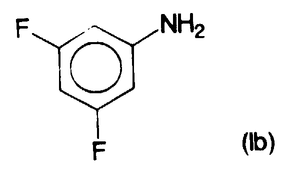 par hydrogénation catalytique d'un composé ou d'un mélange de composés de composés de formule (IIb) 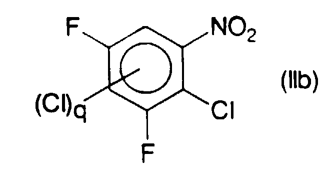 où q vaut de 0 à 2,
choisis parmi le 2-chloro 3,5-difluoronitrobenzène et ses dérivés chlorés en position 4 ou 6, ou en positions 4 et 6.

Le schéma 2 propose deux voies de synthèse multi-étape de la 3,5-difluoroaniline utilisant cette hydrogénation et partant de précurseurs très simples.

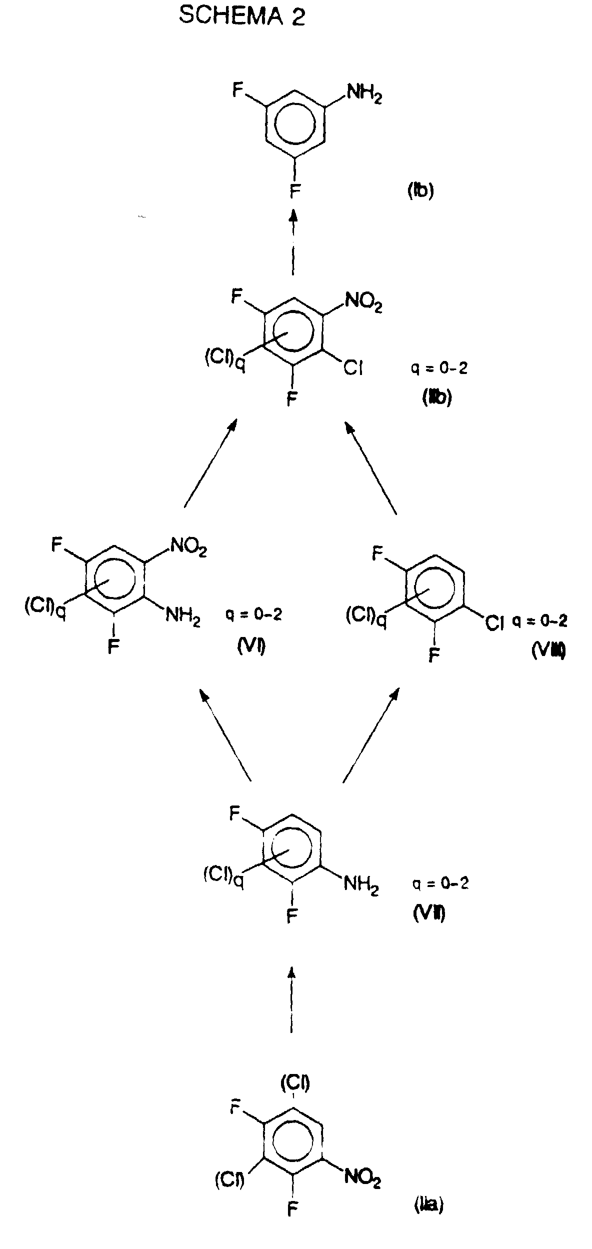

Un composé ou un mélange de composés de formule (IIb) 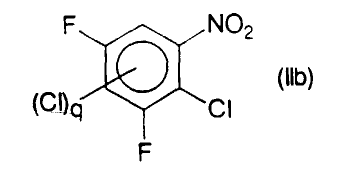 où q vaut de 0 à 2, choisis parmi le 2-chloro 3,5-difluoronitrobenzène et ses dérivés chlorés en position 4 ou 6, ou en positions 4 et 6, peut être obtenu par réaction d'un composé ou d'un mélange de composés de formule (VI) 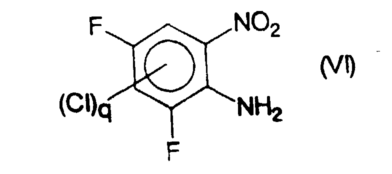 choisis respectivement parmi le 2-amino 3,5-difluoronitrobenzène et ses dérivés chlorés en position 4 ou 6, ou en positions 4 et 6, avec du chlorure cuivreux en présence d'une source d'ions nitrite, notamment d'acide nitreux ou de nitrite de sodium en milieu acide.

Cette réaction, connue sous le nom de réaction de Sandmeyer, réalise une diazotation sur l'atome d'azote de la fonction amine, le groupe azo étant ensuite substitué par un atome de chlore.

Il s'est avéré que cette réaction est particulièrement efficace avec les composés cités ci-dessus et les présents inventeurs ont pu observer des rendements de réaction très élevés, largement supérieurs à 90%.

Le composé ou le mélange de composés de formule (VI) est obtenu par réaction d'un composé ou d'un mélange de composés de formule (VII) 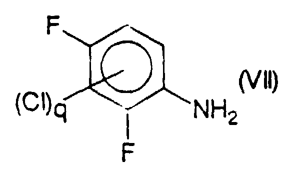 choisis respectivement parmi la 2,4-difluoroaniline et ses dérivés chlorés en position 3 ou 5, ou en positions 3 et 5, avec un réactif de nitration de composés aromatiques, notamment avec de l'acide nitrique.

Un des milieux les meilleurs pour réaliser cette réaction de nitration est un milieu qui comporte de l'acide nitrique, de l'acide sulfurique et un acide carboxylique.

Avantageusement, le mélange réactionnel peut également comporter une proportion notable d'eau qui peut être introduite en tant que telle ou par l'intermédiaire d'une solution d'un des acides minéraux précités.

Avantageusement, la teneur en eau du milieu réactionnel, bien sûr compte non tenu du produit de départ, est comprise entre 1/10éme et une fois la quantité d'acide nitrique pur utilisé.

La quantité d'eau introduite en utilisant un acide nitrique à 65% conduit à des résultats satisfaisants.

De préférence, avant la réaction de nitration, le groupe amino de l'aniline peut être désactivé par acylation. Le groupe acyle peut être installé de façon classique par réaction de l'aniline avec un anhydride ou un halogénure d'acide, ou avec des composés dérivant de l'acide carbonique par mono-amidation.

Il est préférable toutefois que les anilides restent stables dans les conditions de la réaction de nitration.

La réaction du clivage de l'anilide pour redonner l'aniline peut se réaliser dans le même système réactionnel que celui de la nitration. Il suffit pour ce faire de chauffer à une température au moins égale à 50°C, de préférence au moins égale à 80°C, le mélange réactionnel issu de la nitration après addition, facultative, d'une certaine quantité d'eau.

Le produit de la réaction de nitration, avec ou sans passage par l'anilide, conduit à un produit final sous forme de sel de nitroaniline. Ce sel peut être engagé dans la réaction de Sandmeyer au même titre que la nitro aniline elle-même.

En variante, le composé ou le mélange de composés de formule (IIb), peut être avantageusement obtenu en inversant l'ordre des réactions de nitration et de Sandmeyer, à partir de composé(s) de formule (VII).

Ainsi, un composé ou un mélange de composés de formule (IIb) 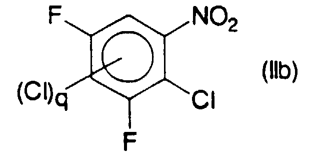 où q vaut de 0 à 2, choisis parmi le 2-chloro 3,5-difluoronitrobenzène et ses dérivés chlorés en position 4 ou 6, ou en positions 4 et 6, peut être obtenu par réaction d'un composé ou d'un mélange de composés de formule (VIII) 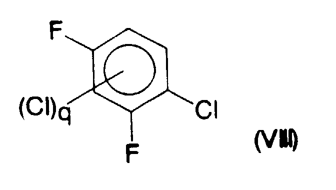 choisis respectivement parmi le 1-chloro 2,4-difluorobenzène et ses dérivés chlorés en position 3 ou 5, ou en positions 3 et 5, avec un réactif de nitration de composés aromatiques, notamment avec de l'acide nitrique.

La nitration en meta des deux atomes de fluor se fait facilement et de façon régiosélective.

Le composé ou le mélange de composés de formule (VIII) est obtenu de préférence en soumettant à la réaction de Sandmeyer un composé ou un mélange de composés de formule (VII) 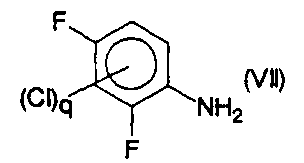 choisis respectivement parmi la 2,4-difluoroaniline et ses dérivés chlorés en position 3 ou 5, ou en positions 3 et 5.

Cette variante est très avantageuse par rapport à l'enchaînement réactionnel expliqué précédemment, puisqu'elle permet d'éviter l'étape de protection de l'amine en présence du groupe nitro par passage à l'anilide, qui engage des réactifs coûteux et qui implique la nécessité de traiter des effluents produits lors de l'application de la réaction de Sandmeyer à la chlorofluoronitroaniline protégée sous forme d'anilide.

Le composé ou le mélange de composés de formule (VII), peut être obtenu avantageusement par hydrogénation d'un composé ou d'un mélange de composés de formule (lla) 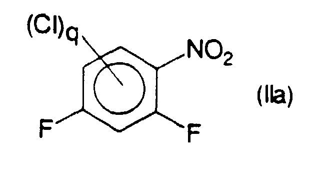 où q vaut de 0 à 2, choisis parmi le 2,4-difluoronitrobenzène et ses dérivés chlorés en position 3 ou 5, ou en positions 3 et 5, notamment suivant un procédé d'hydrogénation catalytique selon l'invention tel que décrit précédemment.

Toute méthode connue de réduction de groupe nitro en amino peut être utilisée sous réserve que cette dernière n'affecte pas les substituants halogène, et surtout fluor.

A cet égard, on peut citer une réduction par hydrogénation catalytique à température ambiante, le catalyseur pouvant être notamment le nickel de Raney ou le palladium.

Il est toutefois préférable d'appliquer les conditions de réduction selon la présente invention, notamment en employant un catalyseur légèrement empoisonné sélectif de la réduction et qui ne permet pas la déchloration.

On peut ainsi préparer avec un bon rendement la 2,4- et la 3,5-difluoroaniline à partir de précurseurs simples, à savoir le 1,3-dichlorobenzène, le 1,2,3- et le 1,2,4-trichlorobenzène, ainsi que le 1,2,3,4-tétrachlorobenzène, ou des mélanges de ces précurseurs puisque tous conduisent au même produit majoritaire final. L'enchaînement des réactions peut se faire en isolant chaque produit intermédiaire à chaque étape. On peut également engager dans une étape le mélange réactionnel brut issu de l'étape précédente. Pour cela, on utilisera un système solvant universel. Le sulfolane convient assez bien. On veillera cependant à ce que les mélanges réactionnels ne contiennent pas de sous-produits, notamment des produits soufrés,susceptibles de constituer ou de générer des poisons pour le ou les catalyseurs des réactions d'hydrogénation.

Ainsi, un procédé de préparation de 2,4-difluoroaniline selon l'invention peut comprendre :
(i) la réaction de 1,2,3-trichlorobenzène avec un réactif de nitration, notamment l'acide nitrique, pour donner du 2,3,4-trichloro nitrobenzène (A),
(ii) la mise en présence de (A) avec du fluorure de potassium pour former le 3-chloro 2,4-difluoronitrobenzène (B),
(iii) l'hydrogénation catalytique de(B) suivant un procédé tel que décrit précédemment.

Ce procédé permet également de synthétiser la 2,4-difluoroaniline en engageant à l'étape (i) du 1,2,4-trichlorobenzène ou du 1,2,3-trichlorobenzène, ou même un mélange d'au moins deux produits choisis parmi le 1,2,3-trichlorobenzène, le 1,2,4-trichlorobenzène et le 1,2,3,4-tétrachlorobenzène. Les produits ou mélanges de produits intermédiaires réagissent avec les mêmes réactifs dans les étapes (ii) et (iii), pour conduire à l'issue de l'étape (iii) à la 2,4-difluoroaniline de façon majoritaire. Les éventuels isomères sous produits, 2,6-difluoroaniline ou 3,5-difluoroaniline, peuvent être aisément séparés par distillation.

De même, un procédé de préparation de 3,5-difluoroaniline selon l'invention peut comprendre la série d'étapes suivantes, ou bien un autre enchaînement mettant en oeuvre une ou plusieurs de ces étapes pour préparer le 2,4-dichloro-3,5-difluoronitrobenzène :
(i) la réaction de 1,2,3-trichlorobenzène avec un réactif de nitration, notamment l'acide nitrique, pour donner du 2,3,4-trichloro nitrobenzène (A),
(ii) la mise en présence de (A) avec du fluorure de potassium pour former le 3-chloro 2,4-difluoronitrobenzène (B),
(iii') l'hydrogénation catalytique de (B) dans des conditions n'affectant pas l'atome de chlore, notamment suivant un procédé tel que décrit précédemment, pour conduire à la 3-chloro 2,4-difluoroaniline (C),
(iv) la réaction de (C) avec un réactif de nitration, conduisant à la 3-chloro 2,4-difluoro 6-nitroaniline (D),
(v) la mise en présence de (D) avec du chlorure cuivreux et une source d'ions nitrites, notamment de l'acide nitreux pour former le 2,4-dichloro 3,5-difluoro nitrobenzène (E),
   et enfin
(vi) l'hydrogénation catalytique de (E) suivant un procédé tel que décrit précédemment.

Ce procédé permet de préparer la 3,5-difluoroaniline en engageant à l'étape (i) du 1,2,4-trichlorobenzène ou du 1,2,3-trichlorobenzène, ou même un mélange d'au moins deux produits choisis parmi le 1,2,3-trichlorobenzène, le 1,2,4-trichlorobenzène et le 1,2,3,4-tétrachlorobenzène. Tous les produits de départ conduisent à l'issue de l'étape (vi) majoritairement à la 3,5-difluoroaniline. Lorsqu'on utilise un mélange de produits de départ, celui-ci conduit quelles que soient ses proportions, à un produit final contenant majoritairement la 3,5-difluoroaniline. Les éventuels isomères sous-produits 2,6-difluoroaniline ou 2,4-difluoroaniline, peuvent être aisément séparés par distillation.

Ce procédé conduit à la 3,5-difluoroaniline avec un rendement élevé. En outre, par rapport aux procédés connus, il présente l'avantage de ne pas faire appel à une réaction de chlorodénitration qui libère NO₂Cl comme sous-produit. Ce sous-produit explosif limitait jusqu'à présent les possibilités de synthèse industrielle de 3,5-difluoroaniline. Le procédé décrit ci-dessus offre une solution rentable et sure au problème de la synthèse industrielle de 3,5-difluoroaniline.

Un procédé du type précité, qui comprend les étapes (i) et (ii), présente l'avantage de pouvoir fabriquer avec un même appareillage industriel aussi bien de la 2,4-difluoroaniline, par simple modification des conditions de l'hydrogénation de l'étape (iii'). Un industriel disposant de cet appareillage polyvalent peut aisément adapter sa production en fonction de la demande.

Un autre procédé de préparation de 3,5-difluoroaniline selon l'invention peut comprendre :
(i) la réaction de 1,2,4-trichlorobenzène, éventuellement de son dérivé chloré en position 3, avec un réactif de nitration, notamment l'acide nitrique,
(ii) la mise en présence du 2,4,5-trichloronitrobenzène, éventuellement chloré en position 3, ainsi obtenu, avec du fluorure de potassium,
(iii) l'hydrogénation catalytique du 5-chloro 2,4-difluoronitrobenzène, éventuellement chloré en position 3, ainsi obtenu, dans des conditions n'affectant pas l'atome de chlore, notamment suivant un procédé tel que décrit précédemment,
(iv) la réaction de la 5-chloro 2,4-difluoro aniline, éventuellement chlorée en position 3, ainsi obtenue, avec du chlorure cuivreux en présence d'une source d'ions nitrite, notamment de l'acide nitreux,
(v) la mise en présence du 1,3-dichloro 4,6-difluoro benzène, éventuellement chloré en position 5, ainsi obtenu, avec un réactif de nitration, notamment de l'acide nitrique, et
(vi) l'hydrogénation catalytique du 2,6-dichloro 3,5-difluoro nitrobenzène, éventuellement chloré en position 4, ainsi obtenu, suivant un procédé tel que décrit précédemment.

Ce procédé, qui, de manière surprenante, ne met en jeu que des réactions à très forte régiosélectivité, permet d'atteindre la 3,5-difluoroaniline avec un très bon rendement et un coût de production compatibles avec une application industrielle. On peut dans ce cas encore utiliser un mélange de produits de départ pour conduire à un produit final largement majoritaire. Grâce à ces procédés, on peut ainsi valoriser des mélanges d'isomères de composés benzéniques chlorés issus de chlorations successives de dichlorobenzène. Ainsi, la monochloration d'ortho-, de méta-, ou de para- dichlorobenzène, qui conduit à un mélange de 1,2,3- et de 1,2,4-trichlorobenzène, qui peuvent encore être substitués par un atome de chlore pour donner du 1,2,3,4-tétrachlorobenzène.

La présente invention va maintenant être illustrée au moyen des exemples suivants.

### EXEMPLES

Exemples 1 à 4 : Réduction du 3-chloro 2,4-difluoro nitrobenzène en 3-chloro 2,4-difluoroaniline.

Tous les tests ont été faits à partir de produits de départ de pureté supérieure à 95 %.

### Exemple 1

Réduction du 3-chloro 2,4-difluoronitrobenzène en 3-chloro 2,4-difluoroaniline.

Dans un réacteur en acier inoxydable de 300 ml agité à 1000 tours/minute, muni d'une pompe pour injection sous pression et d'une capacité (réserve et manomètre détendeur) permettant de contrôler la pression dans le réacteur et de mesurer la quantité d'hydrogène consommé, on place 0,18 g de nickel de Raney dans 100 ml de méthanol.

Le réacteur est purgé trois fois à l'azote puis trois fois à l'hydrogène.

La température du réacteur est portée à 50° C et une pression d'hydrogène de 1,5.10⁶ Pa est installée. Lorsque ces conditions sont établies, on injecte à raison de 28 ml/h, 50 ml d'une solution de 18 g (0,093 mol) de 3-chloro 2,4-difluoronitrobenzène dans le méthanol.

Les conditions de température et de pression sont maintenues constantes pendant toute l'injection, la concentration en 3-chloro 2,4-difluoronitrobenzène étant maintenue par le débit d'injection à une valeur inférieure à 500 ppm.

Après la fin de l'introduction du 3-chloro 2,4-difluoro nitrobenzène, on maintient les conditions d'hydrogénation pendant une heure sans observer de prise d'hydrogène supplémentaire, indiquant que la réaction est terminée.

On sépare, par simple distillation du solvant, 15,3 g d'un produit de couleur très claire contenant 99,5 % de 3-chloro 2,4-difluoro aniline, ne contenant que 0,02 % de 2,4-difluoroaniline.

Le taux de transformation du 3-chloro 2,4-difluoro nitrobenzène est de 100 % et le rendement en 3-chloro 2,4-difluoroaniline est de 99,6 %.

La vitesse de la réaction (disparition du produit de départ) a été d'environ 0,2 mol.h⁻¹.g⁻¹ de catalyseur.

### Exemple 2

On opère dans un réacteur de 300 ml agité par une turbine assurant un excellent transfert gaz-liquide, de telle sorte que le milieu soit toujours saturé en hydrogène dans les conditions de la réaction.

Dans le réacteur on charge 120 ml d'acétate d'éthyle et 0,18 g de nickel de Raney préalablement lavés par de l'éthanol et de l'acétate d'éthyle. Après élimination de l'air par de l'azote puis de l'azote par de l'hydrogène, on pressurise le réacteur sous 1.5.10⁶ Pa absolus avec de l'hydrogène à la température de 50° C. On injecte en 225 mn, une solution de 0,093 mole de 3-chloro 2,4-difluoronitrobenzène dissous dans 80 ml d'acétate d'éthyle.

La consommation d'hydrogène correspond constamment à la stoechiométrie de 3 mole par mole de dérivé nitré.

On vérifie par des moyens analytiques appropriés (mesure de potentiel en continu et contrôle par chromatographie en phase gazeuse CPG) que la concentration en dérivé nitré reste inférieure ou égale à 500 ppm.

Après l'arrêt de l'injection de dérivé nitré, on maintient encore 15 mn les conditions de température et de pression sans constater de consommation d'hydrogène.

Le rendement en 3-chloro 2,4-difluoroaniline (dosé par CPG) est de 99,6 %. Il y a également formation de 0,4 % de 2,4-difluoroaniline résultant de l'hydrodéchloration de la 3-chloro 2,4-difluoroaniline produite.

### Exemple 3

On opère comme dans l'exemple 2 mais dans un mélange eau-méthanol (10-90 en volume) comme solvant et avec du charbon palladié à 10 % de Pd comme catalyseur.

Dans le réacteur on place 100 ml du mélange eau-méthanol et 200 mg de catalyseur.

Le réacteur est pressurisé à 4.10⁵ Pa avec de l'hydrogène et chauffé à 60° C. Le 3-chloro 2,4-difluoronitrobenzène (0,18 mole) est mis en solution dans 70 ml de méthanol et injecté dans le réacteur en 150 mn.

La concentration en dérivé nitré est inférieure à 500 ppm pendant toute la durée de l'hydrogénation.

Le rendement en 3-chloro 2,4-difluoroaniline (dosé par CPG) est de 88,5 %. Il y a également 10,2 % de 2,4-difluoroaniline résultant de l'hydrodéchloration de la 3-chloro 2,4-difluoroaniline produite.

Cet exemple montre que le charbon palladié est un catalyseur susceptible de catalyser à la fois la réduction du groupe nitro et la substitution des atomes de chlore par l'hydrogène.

L'analyse des fluorures dans le milieu liquide et dans le catalyseur montre que l'hydrodéfluoration est inférieure à 0,1 % par rapport au dérivé nitré engagé.

### Exemple 4

On opère comme dans l'exemple 2 avec des charges et des conditions différentes.

Dans la réacteur on place 180 ml d'un mélange méthanol-eau (dans un rapport volumique 66/34) et 300 mg de nickel de Raney. On pressurise le réacteur à 2.10⁶ Pa avec de l'hydrogène et on chauffe à 60° C. On maintient alors la pression constante à 2,2.10⁶ Pa.

On injecte en 270 mn une solution de 0,155 mole de 3-chloro 2,4-difluoro nitrobenzène dans 70 ml de méthanol.

Le rendement en 3-chloro 2,4-difluoroaniline est de 87 % et le rendement en 2,4-difluoroaniline est de 13 %.

### Exemples comparatifs 1 à 3

L'hydrogénation de 3-chloro 2,4-difluoro nitrobenzène est réalisée en plaçant la totalité du composé benzénique nitré dans le réacteur au début de la réaction.

### Exemple comparatif 1

Dans un autoclave en acier inoxydable agité par secousses, équipé d'une capacité permettant de contrôler la pression dans l'autoclave et de mesurer la quantité d'hydrogène consommé, on place 10 g de 3-chloro 2,4-difluoronitrobenzène (0,049 mol) dans 38 ml de méthanol, avec 13 ml de chlorobenzène, 0,8 ml d'eau et 0,08 g de charbon platiné à 5 % de platine. Le chlorobenzène est utilisé pour éviter toute déshalogénation intempestive.

L'autoclave est purgé trois fois à l'azote puis trois fois à l'hydrogène.

La température de l'autoclave est portée à 35° C et une pression d'hydrogène de 4,2.10⁶ Pa est installée. La pression est maintenue à cette valeur par introduction d'hydrogène en continu, alors que la température s'élève à 50° C à cause de l'exothermicité de la réaction. La réaction est terminée en 45 minutes.

Après cette période, on vérifie que le taux de transformation du 3-chloro 2,4-difluoronitrobenzène de départ est de 100 %. On mesure de même le taux de formation de 3-chloro 2,4-difluoroaniline qui vaut 81 %, le reste du produit de transformation étant constitué par des produits divers dont des composés benzéniques nitrés halogénés à fonctions azo ou azoxy, ayant perdu éventuellement du chlore et du fluor. La 3-chloro 2,4-difluoroaniline peut être isolée par distillation, puis soumise à une salification sous forme de chlorhydrate et nouvelle distillation. Ces opérations de purification font toutefois chuter le rendement à environ 50 %.

La vitesse de la réaction a été de 0,83 mol.h⁻¹.g⁻¹ de catalyseur.

### Exemple comparatif 2

Dans un autoclave en acier inoxydable agité par secousses, équipé d'une capacité permettant de contrôler la pression dans l'autoclave et de mesurer la quantité d'hydrogène consommé, on place 3,7 g de 3-chloro 2,4-difluoronitrobenzène (0,018 mol) dans 100 ml d'éthanol à 4 % d'eau, avec 30 mg de charbon palladié à 10 % de Pd.

La température de l'autoclave est portée à 35° C et une pression d'hydrogène de 1.10⁵ Pa est installée. La pression et la température sont maintenues pendant 400 minutes.

Après cette période de chauffage, on vérifie que le taux de transformation du 3-chloro 2,4-difluoronitrobenzène de départ est de 100 %. On mesure de même le taux de formation de 3-chloro 2,4-difluoroaniline qui vaut 84 %, le reste du produit de transformation étant constitué des mêmes produits que dans l'exemple comparatif 1.

La vitesse de la réaction a été voisine de 0,1 mol.h⁻¹.g⁻¹ de catalyseur.

### Exemple comparatif 3

On répète l'exemple comparatif 2 avec 90 mg de catalyseur et 14,8 g de 3-chloro 2,4-difluoronitrobenzène.

La durée de la réaction est de 400 mn. Le taux de transformation du dérivé nitré est de 100 %. Le rendement en 3-chloro 2,4-difluoroaniline est de 57 %. Le reste est toujours constitué des mêmes sous-produits que dans les exemples comparatifs 1 et 2.

### Exemple comparatif 4 (vis-à-vis de l'exemple 3)

Dans cet exemple comparatif, l'hydrogénation est réalisée en introduisant le 3-chloro 2,4-difluoronitrobenzène de façon progressive mais sans respecter la condition selon laquelle la teneur en 3-chloro 2,4-difluoronitrobenzène dans le milieu réactionnel reste inférieure à 1000 ppm.

Dans un réacteur en acier inoxydable de 300 ml agité à 1000 tours/minute, muni d'une pompe pour injection sous pression et d'une capacité (réserve et manomètre détendeur) permettant de contrôler la pression dans le réacteur et de mesurer la quantité d'hydrogène consommé, on place 0,20 g de charbon palladié à 10 % de Pd dans 90 ml de méthanol et 10 ml d'eau.

Le réacteur est purgé trois fois à l'azote puis trois fois à l'hydrogène.

La température du réacteur est portée à 60° C et une pression d'hydrogène de 3.10⁵ Pa est installée. Lorsque ces conditions sont établies, on injecte à raison de 28 ml/h, 50 ml d'une solution de 39 g (0,2 mol) de 3-chloro 2,4-difluoronitrobenzène dans le méthanol.

Les conditions de température et de pression sont maintenues constantes pendant toute l'injection.

Après la fin de l'introduction du 3-chloro 2,4-difluoro nitrobenzène, on maintient les conditions d'hydrogénation pendant une heure sans observer de prise d'hydrogène supplémentaire, indiquant ainsi que la réaction est terminée.

On sépare, par simple distillation du solvant, un produit de couleur claire.

On mesure le rendement total de formation d'anilines qui vaut 88 %.

Le taux de transformation du 3-chloro 2,4-difluoronitrobenzène est toujours de 100 %.

L'exemple 1 confronté aux exemples comparatifs 1, 2 et 3, met bien en évidence l'amélioration remarquable du rendement de la réaction d'hydrogénation réductrice grâce à l'injection du produit de départ de façon progressive en maintenant une concentration en composé nitré inférieure à 1000 ppm.

### Exemples 5 à 8 : Hydrodéshalogénation de la 3-chloro 2,4-difluoroaniline pour former la 2,4-difluoroaniline (hydrodéchloration).

### Exemple 5

Dans une bombe agitée (autoclave) de 125 ml, commercialisée par la Société PROLABO, agitée par secousses, on charge 1,64 g de 3-chloro 2,4-difluoroaniline de l'exemple 1, 0,05 g de charbon palladié à 10 % de Pd dans 22 ml de méthanol, avec 11 ml d'une solution de soude à 1 mol/l (0,011 mol).

L'autoclave fermé est purgé trois fois avec de l'azote à une pression de 3.10⁵ Pa puis trois fois avec de l'hydrogène à une pression de 3.10⁵ Pa.

La pression dans l'autoclave est portée à 2.10⁶ Pa d'hydrogène et la température est élevée à 90° C en 25 minutes. Ces conditions sont maintenues pendant 3 heures.

Au bout de ce temps, le taux de transformation de la 3-chloro 2,4-difluoroaniline est déterminé par chromatographie en phase gazeuse à 98 %.

Le rendement en 2,4-difluoroaniline est de 97,5 %.

La vitesse de la réaction a été de 0,065 mol.h⁻¹.g⁻¹ de catalyseur.

Le rendement global des exemples 1 et 2 pour la préparation de 2,4-difluoroaniline par hydrogénation catalytique à partir de 3-chloro 2,4-difluoronitrobenzène est de 97,1 %.

### Exemple 6

Dans un réacteur agité par une turbine assurant dans les conditions de la réaction un transfert d'hydrogène gaz/liquide tel que le milieu soit toujours saturé en hydrogène, on charge 44 ml de méthanol, 22 ml d'eau, 0,1 g de nickel de Raney, 0,022 mole d'hydroxyde de sodium et 0,02 mole de 3-chloro 2,4-difluoroaniline.

On chauffe à 90° C sous pression d'hydrogène qu'on maintient constante à 7,5.10⁵ Pa absolu à 90° C. La réaction est terminée au bout de 4 heures.

Le taux de transformation final de la 3-chloro 2,4-difluoro aniline est de 75,5 % . Le rendement en 2,4-difluoroaniline est de 80 % par rapport au produit de transformation et il y a 20 % d'aniline.

Le nickel de Raney est peu efficace pour réaliser la réaction d'hydrodéchloration et de surcroît catalyse de façon importante l'hydrodéfluoration de la 2,4-difluoroaniline dans ces conditions.

### Exemple 7

Dans le même réacteur que celui de l'exemple 6,on charge 88 ml de méthanol, 44 ml d'eau, 0,2 g de charbon palladié à 10 % Pd et 0,04 mol de 3-chloro 2,4-difluoro aniline.

On installe une pression d'hydrogène dans le réacteur que l'on chauffe à 90° C. La pression d'hydrogène est maintenue constante à 1,9.10⁶ Pa absolus à 90° C. Au cours de la réaction, on ajoute de la soude au fur et à mesure du dégagement d'acide chlorhydrique jusqu'à une quantité comparable à celle utilisée pour une quantité correspondante de chlorodifluoroaniline dans l'exemple 6.

Après une durée de 3 heures, le taux de transformation de la 3-chloro 2,4-difluoro aniline est de 100 % et le rendement en 2,4-difluoroaniline est de 99 %. Le reste du produit de transformation (1 %) est constitué de 4-fluoro aniline et de traces d'aniline.

### Exemple 8

On répète l'exemple 7 à 125° C sous 2.10⁶ Pa d'hydrogène. Après 2 heures, le rendement en 2,4-difluoroaniline est de 99,2 % et celui de la 4-fluoroaniline est de 0,8 %.

### Exemple 9 : Hydrogénation du 3-chloro 2,4-difluoronitrobenzène en 2,4-difluoroaniline dans un seul réacteur enchaînant les réactions de réduction puis d'hydrodéchloration.

Dans le réacteur décrit à l'exemple 1, on charge 160 ml de méthanol et 30 ml d'eau ainsi que 0,2 g de charbon palladié à 10 % Pd. On injecte en 5 heures une solution de 0,207 mole de 3-chloro 2,4-difluoro nitrobenzène dans 100 ml de méthanol. Le réacteur est pressurisé sous 2.10⁶ Pa maintenus constants et sa température est de 60° C. L'hydrogénation étant terminée au bout de cette période, on ajoute 0,22 mole de soude sous forme de solution aqueuse concentrée et on chauffe à 120° C en maintenant la pression à 2.10⁶ Pa d'hydrogène.

Après 5 heures de réaction, on mesure par chromatographie en phase gazeuse que le taux de transformation du 3-chloro 2,4-difluoro nitrobenzène engagé est de 100 % et le taux de 2,4-difluoro aniline est de 95 % par rapport au 3-chloro 2,4-difluoro nitrobenzène engagé. La 2,4-difluoro aniline peut être isolée aisément du milieu réactionnel par distillation.

### Exemple 10 : Hydrodéchloration de la 2,6-dichloro 3,5-difluoroaniline en 3,5-difluoroaniline en présence de soude.

Dans un réacteur de 150 ml, on charge 30 g d'une solution de 2,6-dichloro 3,5-difluoroaniline à 30 % en poids environ dans un mélange méthanol/eau 13/1 en poids. On ajoute 0,25 g de catalyseur charbon palladié à 10 % de Pd et 12 g de solution de soude à 30 %. On purge le réacteur sous azote puis sous hydrogène. On porte le réacteur à 120°C sous 2.10⁶ Pa d'hydrogène. La réaction est terminée au bout de 2 heures : le taux de transformation est de 100 % et le rendement de transformation en 3,5-difluoroaniline est de 90 %.

Le reste du produit de transformation comprend de la fluorométhoxyaniline formée par substitution nucléophile aromatique dans le milieu méthanolique basique.

Cette réaction parasite peut être éliminée en utilisant une base moins forte comme le montre l'exemple suivant.

### Exemple 11 : Hydrodéchloration de la 2,6-dichloro 3,5-difluoroaniline en 3,5-difluoroaniline en présence de magnésie.

Dans un réacteur de 150 ml, on charge 45 g d'une solution de 2,6-dichloro 3,5-difluoroaniline à 20 % en poids dans un mélange méthanol/eau 14/1 en poids. On ajoute 0,25 g de catalyseur charbon palladié à 10% Pd et 18 g de suspension aqueuse de magnésie à 10 % (MgO). On purge le réacteur à l'azote puis à l'hydrogène puis on porte le réacteur à 120°C sous 2.10⁶ Pa avec l'hydrogène. On arrête la réaction au bout de 6 heures : le taux de transformation est de 97,2 %, le rendement de transformation en 3,5-difluoroaniline est de 80,1 %. L'autre produit de transformation est le dérive encore monochloré (2-chloro 3,5-diluoroaniline) que l'on peut transformer en 3,5-difluoroaniline en poursuivant l'hydrogénation. Il n'y a pas dans ce cas de réaction parasite de substitution nucléophile aromatique due au solvant, grâce à la faible basicité de la magnésie. Les conditions de température permettent de travailler en présence d'une suspension de magnésie sans aucun problème.

## Revendications

1. Procédé pour la préparation d'un composé de formule (I) 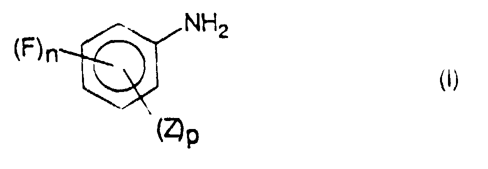 où
n vaut de 1 à 5,
Z est un radical stable dans des conditions d'hydrogénation catalytique,
p est inférieur à 5-n,
selon lequel on soumet un composé de formule (II) 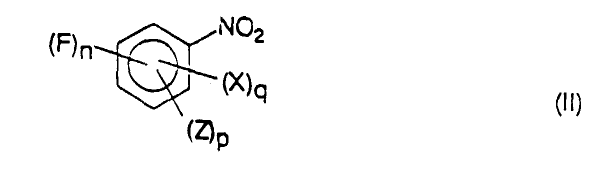 où
n, Z et p ont les significations précédentes,
X est un radical hydrogénolysable,
q vaut de 0 à 5-(n + p),
à une hydrogénation catalytique dans un milieu liquide contenant un catalyseur, sous pression d'hydrogène, réalisant une réaction de réduction catalytique et, le cas échéant, une réaction d'hydrogénolyse,
caractérisé en ce que le composé de formule (Il) est introduit progressivement dans ledit milieu de sorte que la teneur en composé de formule (II) dans le liquide reste inférieure ou égale à 1000 ppm en masse, pour former sélectivement le composé de formule (I).

2. Procédé selon la revendication 1, caractérisé en ce que le composé de formule (II) est introduit progressivement dans ledit milieu de sorte que la teneur en composé de formule (II) dans le liquide reste inférieure ou égale à 500 ppm en masse, notamment dans la gamme de 200 à 500 ppm.

3. Procédé selon la revendication 1 ou 2, caractérisé en ce que X est un atome de chlore et q vaut de 1 à 5-(n+ p),
et
les conditions d'hydrogénation sont adaptées pour former dans une étape a) un composé de formule (III) 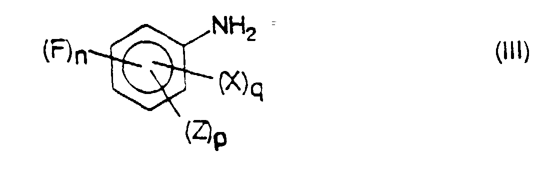 à partir du composé de formule (II) par réduction catalytique, puis
dans une étape b) former le composé de formule (I) à partir du composé de formule (III) par hydrogénolyse.

4. Procédé selon l'une des revendications 1 à 3, caractérisé en ce que le catalyseur est le même pendant toute la durée de l'hydrogénation et comprend du charbon palladié.

5. Procédé selon la revendication 3, caractérisé en ce que le catalyseur de l'étape a) est choisi parmi le nickel de Raney et les catalyseurs métalliques empoisonnés, tels que le nickel de Raney empoisonné, et le catalyseur de l'étape b) n'est pas choisi parmi le nickel de Raney et les catalyseurs métalliques empoisonnés, tels que le nickel de Raney empoisonné.

6. Procédé selon l'une des revendications 1 à 5, caractérisé en ce que ledit liquide est un solvant polaire, pouvant être protique ou aprotique.

7. Procédé selon l'une des revendications 1 à 6, caractérisé en ce que la température de réaction est de 0 à 80° C, pendant toute l'hydrogénation si q = 0, ou pendant l'étape (a) si q > 0.

8. Procédé selon l'une des revendications 1 à 7, caractérisé en ce que le pression partielle d'hydrogène est de 10⁵ à 5.10⁶ Pa pendant toute l'hydrogénation si q = 0, ou pendant l'étape (a) si q> 0.

9. Procédé selon la revendication 4, dans lequel ledit milieu contient une base, notamment une base alcaline.

10. Procédé selon la revendication 4, caractérisé en ce que l'on introduit une base, notamment une base alcaline, dans le milieu à l'étape (b).

11. Procédé selon l'une des revendications 9 ou 10, caractérisé en ce que ladite base est de la soude, présente dans le liquide réactionnel en une concentration inférieure ou égale à 1 ml/l.

12. Procédé selon la revendication 4, caractérisé en ce que la température de réaction à l'étape (b) est de 80 à 150° C.

13. Procédé selon la revendication 4, caractérisé en ce que la pression partielle d'hydrogène à l'étape (b) est de 1,5.10⁶ à 5.10⁶ Pa.

14. Procédé selon l'une des revendications 1 à 13, pour la préparation de 2,4-difluoroaniline de formule (la), 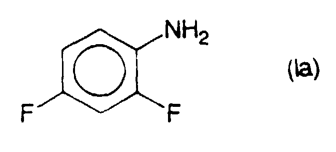 caractérisé en ce que l'on soumet à l'hydrogénation catalytique un composé ou mélange de composés de formule (IIa) 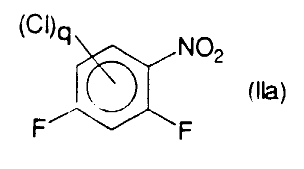 où q vaut de 0 à 2, choisis parmi le 2,4-difluoronitrobenzène et ses dérivés chlorés en position 3 ou 5, ou en positions 3 et 5.

15. Procédé selon la revendication 14, caractérisé en ce que le composé ou le mélange de composés de formule (lla) est obtenu par réaction d'un composé ou d'un mélange de composés de formule (IV) 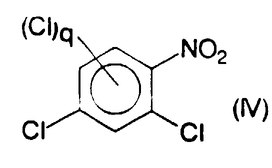 choisis respectivement parmi le 2,4-dichloronitrobenzène, et ses dérivés chlorés en position 3 ou 5, ou en positions 3 et 5, avec un fluorure alcalin, notamment du fluorure de potassium, dans un solvant aprotique polaire.

16. Procédé selon la revendication 15, caractérisé en ce que le composé ou le mélange de composés de formule (IV) est obtenu par réaction d'un composé ou d'un mélange de composés de formule (V) 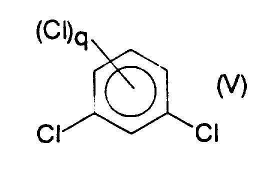 choisis respectivement parmi le 1 ,3-dichlorobenzène, et ses dérivés chlorés en position 2 ou 4, ou en positions 2 et 4, avec un réactif de nitration de composés aromatiques, notamment avec de l'acide nitrique.

17. Procédé selon l'une des revendications 1 à 13, pour la préparation de 3,5-difluoroaniline de formule (Ib), 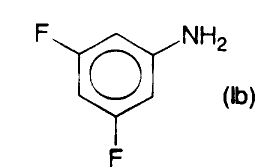 caractérisé en ce que l'on soumet à l'hydrogénation catalytique un composé ou un mélange de composés de formule (IIb) 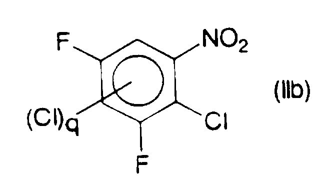 où q vaut de 0 à 2, choisis parmi le 2--chloro 3,5-difluoronitrobenzène, et ses dérivés chlorés en position 4 ou 6, ou en positions 4 et 6.

18. Procédé selon la revendication 17, caractérisé en ce que le composé ou le mélange de composés de formule (IIb) est obtenu par réaction d'un composé ou d'un mélange de composés de formule (VI) 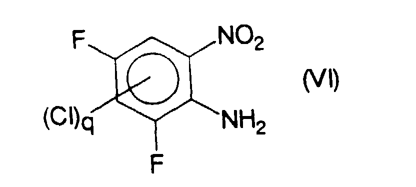 choisis respectivement parmi le 2-amino 3,5-difluoronitrobenzène, et ses dérivés chlorés en position 4 ou 6, ou en positions 4 et 6, avec du chlorure cuivreux en présence d'acide nitreux.

19. Procédé selon la revendication 18, caractérisé en ce que le composé ou le mélange de composés de formule (VI) est obtenu par réaction d'un composé ou d'un mélange de composés de formule (VII) 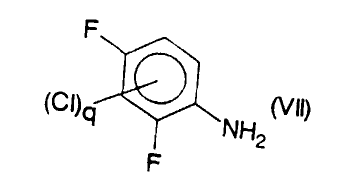 choisis respectivement parmi la 2,4-difluoroaniline et ses dérivés chlorés en position 3 ou 5, ou en positions 3 et 5, avec un réactif de nitration de composés aromatiques, notamment avec de l'acide nitrique.

20. Procédé selon la revendication 17, caractérisé en ce que le composé ou le mélange de composés de formule (IIb) est obtenu par réaction d'un composé ou d'un mélange de composés de formule (VIII) 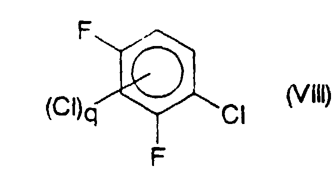 choisis respectivement parmi le 1-chloro 2,4-difluorobenzène et ses dérivés chlorés en position 3 ou 5, ou en positions 3 et 5, avec un réactif de nitration de composés aromatiques, notamment avec de l'acide nitrique.

21. Procédé selon la revendication 20, caractérisé en ce que le composé ou le mélange de composés de formule (VIII) est obtenu par réaction d'un composé ou d'un mélange de composés de formule (VII) 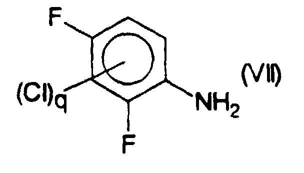 choisis respectivement parmi la 2,4-difluoroaniline, et ses dérivés chlorés en position 3 ou 5, ou en positions 3 et 5, avec du chlorure cuivreux en présence d'acide nitreux.

22. Procédé selon la revendication 19 ou 21, caractérisé en ce que le composé ou le mélange de composés de formule (VII) est obtenu par hydrogénation d'un composé ou mélange de composés de formule (IIa) 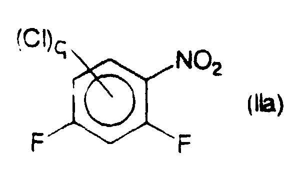 où q vaut de 0 à 2, choisis parmi le 2,4-difluoronitrobenzène et ses dérivés chlorés en position 3 ou 5, ou en positions 3 et 5, notamment suivant un procédé selon l'une quelconque des revendications 1 à 14.

## Patentansprüche

1. Verfahren zur Herstellung einer Verbindung der Formel (I) 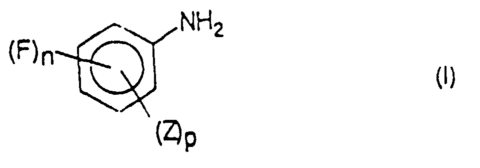
in der n = 1 bis 5,
Z ein unter den Bedingungen einer katalytischen Hydrierung beständiges Radikal und
p kleiner als 5 - n ist,
wobei man eine Verbindung der Formel (II), 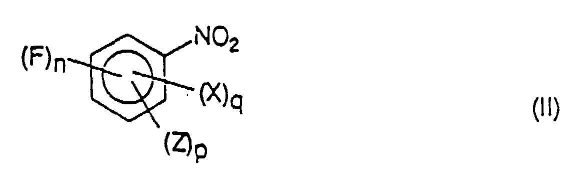 in der n, Z und p die vorstehende Bedeutung haben,
X ein hydrogenolysierbares Radikal und
q = 0 bis 5-(n + p) ist,
unter Wasserstoffdruck einer katalytischen Hydrierung in einer einen Katalysator enthaltenden Flüssigkeit unterzieht, bei der eine katalytische Reduktion und gegebenenfalls eine Hydrogenolyse erfolgt, dadurch gekennzeichnet, daß die Verbindung der Formel (II) nach und nach in das besagte Milieu gegeben wird, so daß der Gehalt an der Verbindung der Formel (II) in der Flüssigkeit kleiner oder gleich 1000 ppm bleibt, um selektiv die Verbindung der Formel (I) zu bilden.

2. Verfahren nach Anspruch 1, dadurch gekennzeichnet, daß die Verbindung der Formel (II) nach und nach in das besagte Milieu gegeben wird, so daß der Gehalt an der Verbindung der Formel (II) in der Flüssigkeit kleiner oder gleich 500 ppm bleibt, vor allem im Bereich von 200 bis 500 ppm.

3. Verfahren nach Anspruch 1 oder 2, dadurch gekennzeichnet, daß X ein Chloratom und q = 1 bis 5 - (n + p) ist, und die Hydrierbedingungen so angepaßt werden, daß in einer Stufe (a) ausgehend von der Verbindung der Formel (II) durch katalytische Reduktion eine Verbindung der Formel (III) 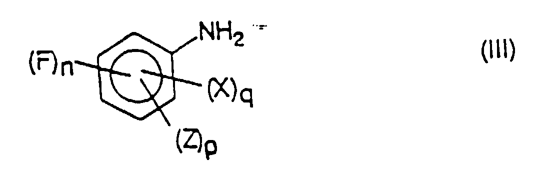 gebildet wird, und in einer anschließenden Stufe (b) ausgehend von der Verbindung der Formel (III) durch Hydrogenolyse die Verbindung der Formel (I) entsteht.

4. Verfahren nach einem der Ansprüche 1 bis 3, dadurch gekennzeichnet, daß der Katalysator während der gesamten Dauer der Hydrierung der gleiche ist und Palladiumkohle enthält.

5. Verfahren nach Anspruch 3, dadurch gekennzeichnet, daß der Katalysator der Stufe (a) unter Raneynickel und vergifteten Metallkatalysatoren, wie etwa vergiftetem Raneynickel ausgewählt wird, und der Katalysator der Stufe (b) unter Raneynickel und vergifteten Metallkatalysatoren wie etwa vergiftetem Raneynickel nicht ausgewählt wird.

6. Verfahren nach einem der Ansprüche 1 bis 5, dadurch gekennzeichnet, daß die besagte Flüssigkeit ein polares Lösungsmittel ist, das protisch oder aprotisch sein kann.

7. Verfahren nach einem der Ansprüche 1 bis 6, dadurch gekennzeichnet, daß die Reaktionstemperatur während der gesamten Hydrierung 0 bis 80°C beträgt, wenn q = 0, oder während der Stufe (a), wenn q > 0.

8. Verfahren nach einem der Ansprüche 1 bis 7, dadurch gekennzeichnet, daß der Wasserstoff-Partialdruck während der gesamten Hydrierung 10⁵ bis 5·10⁶ Pa beträgt, wenn q = 0, oder während der Stufe (a), wenn q > 0.

9. Verfahren nach Anspruch 4, bei dem das besagte Milieu eine Base, besonders eine Alkalibase enthält.

10. Verfahren nach Anspruch 4, dadurch gekennzeichnet, daß man in das Milieu der Stufe (b) eine Base, besonders eine Alkalibase gibt.

11. Verfahren nach einem der Ansprüche 9 oder 10, dadurch gekennzeichnet, daß es sich bei der besagten Base um Natronlauge handelt, die in der Reaktionsflüssigkeit in einer Konzentration kleiner oder gleich 1 ml/l vorliegt.

12. Verfahren nach Anspruch 4, dadurch gekennzeichnet, daß die Reaktionstemperatur der Stufe (b) 80 bis 150°C beträgt.

13. Verfahren nach Anspruch 4, dadurch gekennzeichnet, daß der Wasserstoff-Partialdruck in der Stufe (b) 1,5·10⁶ bis 5·10⁶ Pa beträgt.

14. Verfahren nach einem der Ansprüche 1 bis 13 zur Herstellung von 2,4-Difluoranilin der Formel (Ia), 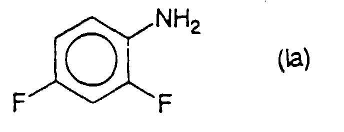 dadurch gekennzeichnet, daß man eine katalytische Hydrierung durchführt bei einer Verbindung oder Mischung von Verbindungen der Formel (IIa), 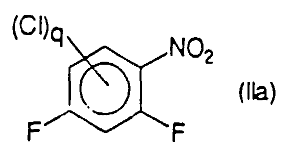 in der q = 0 bis 2 ist, und die man unter 2,4-Difluornitrobenzol und seinen in Position 3 oder 5 oder in Position 3 und 5 chlorierten Derivaten auswählt.

15. Verfahren nach Anspruch 14, dadurch gekennzeichnet, daß die Verbindung oder die Mischung aus Verbindungen der Formel (IIa) durch Reaktion einer Verbindung oder einer Mischung aus Verbindungen der Formel (IV) 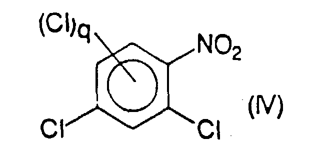 mit einem Alkalifluorid, besonders Kaliumfluorid, in einem polaren aprotischen Lösungsmittel erhalten wird, die man jeweils zwischen 2,4-Dichlornitrobenzol und seinen in Position 3 oder 5, oder in Position 3 und 5 chlorierten Derivaten auswählt.

16. Verfahren nach Anspruch 15, dadurch gekennzeichnet, daß die Verbindung oder die Mischung aus Verbindungen der Formel (IV) durch Reaktion einer Verbindung oder einer Mischung aus Verbindungen der Formel (V), 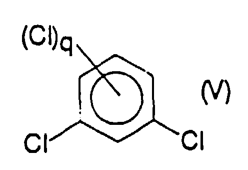 mit einem Reagenz für die Nitrierung aromatischer Verbindungen, besonders mit Salpetersäure erhalten wird, die man jeweils unter 1,3-Dichlorbenzol und seinen in Position 2 oder 4 oder in Position 2 und 4 chlorierten Derivaten auswählt.

17. Verfahren nach einem der Ansprüche 1 bis 13 für die Herstellung von 3,5-Difluoranilin der Formel (Ib), 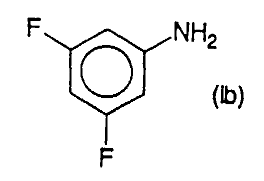 dadurch gekennzeichnet, daß man eine Verbindung oder eine Mischung aus Verbindungen der Formel (IIb) 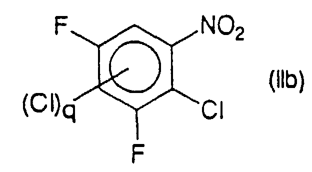 in der q = 0 bis 2 ist, und die man unter 2-Chlor-3,5-difluornitrobenzol und seinen in Position 4 oder 6 oder in Position 4 und 6 chlorierten Derivaten auswählt, der katalytischen Hydrolyse unterzieht.

18. Verfahren nach Anspruch 17, dadurch gekennzeichnet, daß die Verbindung oder die Mischung aus Verbindungen der Formel (IIb) durch Reaktion einer Verbindung oder einer Mischung aus Verbindungen der Formel (VI) 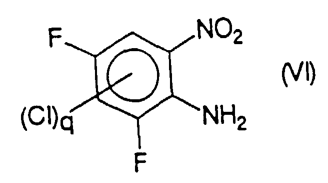 mit Kupfer(I)-chlorid in Gegenwart von salpetriger Säure erhalten wird, die man jeweils unter 2-Amino-3,5-difluornitrobenzol und seinen in Position 4 oder 6 oder in Position 4 und 6 chlorierten Derivaten auswählt.

19. Verfahren nach Anspruch 18, dadurch gekennzeichnet, daß die Verbindung oder die Mischung aus Verbindungen der Formel (VI) durch Reaktion einer Verbindung oder einer Mischung aus Verbindungen der Formel (VII), 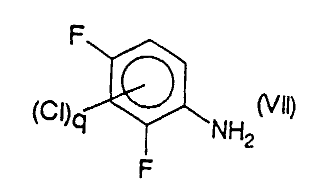 mit einem Reagenz für die Nitrierung aromatischer Verbindungen, besonders mit Salpetersäure erhalten wird, die man jeweils unter 2,4-Difluoranilin und seinen in Position 3 oder 5 oder in Position 3 und 5 chlorierten Derivaten auswählt.

20. Verfahren nach Anspruch 17, dadurch gekennzeichnet, daß die Verbindung oder die Mischung aus Verbindungen der Formel (IIb) durch Reaktion einer Verbindung oder Mischung aus Verbindungen der Formel (VIII), 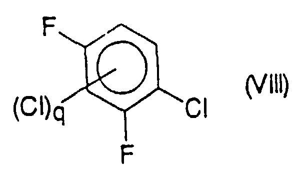 mit einem Reagenz für die Nitrierung aromatischer Verbindungen, besonders mit Salpetersäure erhalten wird, die man jeweils unter 1-Chlor-2,4-difluorbenzol und seinen in Position 3 oder 5 oder in Position 3 und 5 chlorierten Derivaten auswählt.

21. Verfahren nach Anspruch 20, dadurch gekennzeichnet, daß die Verbindung oder die Mischung aus Verbindungen der Formel (VIII) durch Reaktion einer Verbindung oder einer Mischung aus Verbindungen der Formel (VII), 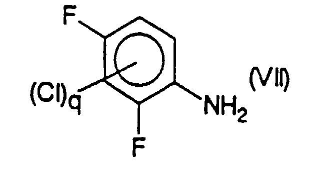 mit Kupfer(I)-chlorid in Gegenwart von salpetriger Säure erhalten wird, die man jeweils unter 2,4-Difluoranilin und seinen in Position 3 oder 5 oder in Position 3 und 5 chlorierten Derivaten auswählt.

22. Verfahren nach Anspruch 19 oder 21, dadurch gekennzeichnet, daß die Verbindung oder die Mischung aus Verbindungen der Formel (VII) durch Hydrierung einer Verbindung oder einer Mischung aus Verbindungen der Formel (IIa), 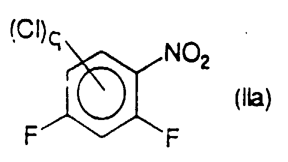 erhalten wird, in der q = 0 bis 2 ist, und die man unter 2,4-Difluornitrobenzol und seinen in Position 3 oder 5 oder in Position 3 und 5 chlorierten Derivaten auswählt, besonders gemäß einem Verfahren nach irgendeinem der Ansprüche 1 bis 14.

## Claims

1. Process for preparing a compound of formula (I) 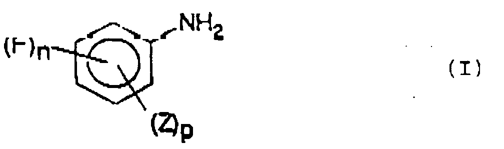 wherein
n is equal to 1 to 5,
Z is a radical which is stable under catalytic hydrogenation conditions,
p is less than 5-n,
wherein a compound of formula (II) 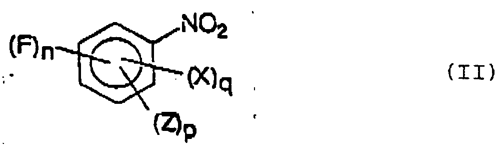 wherein
n, z and p are as hereinbefore defined,
X is a radical capable of hydrogenolysis,
q equals 0 to 5-(n + p),
is subjected to catalytic hydrogenation in a liquid medium containing a catalyst, under hydrogen pressure, carrying out a catalytic reduction reaction and, in certain cases, a hydrogenolysis reaction,
characterised in that the compound of formula (II) is added progressively to said medium, so that the content of the compound of formula (II) in the liquid remains less than or equal to 1000 ppm by mass, in order to form selectively the compound of formula (I).

2. Process according to claim 1, characterised in that the compound of formula (II) is added progressively to said medium so that the content of the compound of formula (II) in the liquid remains less than or equal to 500 ppm by mass, more particularly within the range from 200 to 500 ppm.

3. Process according to claim 1 or 2, characterised in that X is a chlorine atom and q equals 1 to 5-(n + p),
and
the hydrogenation conditions are adapted so as to form in a step a) a compound of formula (III) 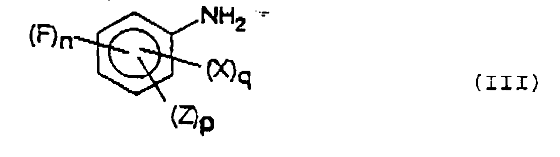 from the compound of formula (II) by catalytic reduction, then
in a step b) to form the compound of formula (I) from the compound of formula (III) by hydrogenolysis.

4. Process according to one of claims 1 to 3, characterised in that the catalyst remains the same throughout the entire duration of the hydrogenation and comprises palladium on charcoal.

5. Process according to claim 3, characterised in that the catalyst in step a) is selected from Raney nickel and the poisoned metal catalysts such as poisoned Raney nickel, and the catalyst in step b) is not selected from Raney nickel and the poisoned metal catalysts such as poisoned Raney nickel.

6. Process according to one of claims 1 to 5, characterised in that the liquid is a polar solvent which may be protic or aprotic.

7. Process according to one of claims 1 to 6, characterised in that the reaction temperature is from 0 to 80°C, throughout the entire hydrogenation if q = 0 or throughout step (a) if q > 0.

8. Process according to one of claims 1 to 7, characterised in that the partial hydrogen pressure is from 10⁵ to 5x10⁶ Pa throughout the entire hydrogenation if q = 0 or throughout step (a) if q > 0.

9. Process according to claim 4, wherein said medium contains a base, notably an alkaline base.

10. Process according to claim 4, characterised in that a base, notably an alkaline base, is added to the medium in step (b).

11. Process according to one of claims 9 and 10, characterised in that the base is sodium hydroxide which is present in the reaction liquid in a concentration less than or equal to 1 ml/l.

12. Process according to claim 4, characterised in that the reaction temperature in step (b) is 80 to 150°C.

13. Process according to claim 4, characterised in that the partial hydrogen pressure in step (b) is from 1.5x10⁶ to 5x10⁶ Pa.

14. Process according to one of claims 1 to 13 for preparing 2,4-difluoroaniline of formula (Ia) 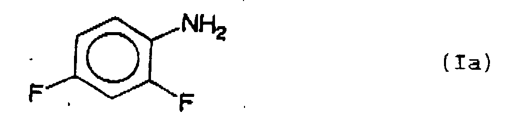 characterised in that a compound or mixture of compounds of formula (IIa) 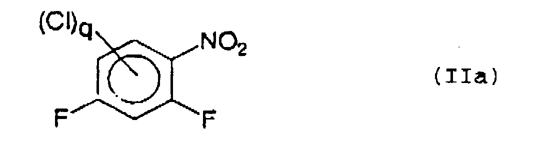 wherein q equals 0 to 2, which are selected from 2,4-difluoronitrobenzene and its derivatives chlorinated in position 3 or 5 or in positions 3 and 5, is subjected to catalytic hydrogenation.

15. Process according to claim 14, characterised in that the compound or mixture of compounds of formula (IIa) is obtained by reacting a compound or mixture of compounds of formula (IV) 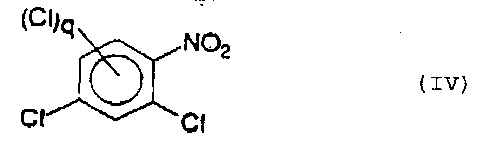 which are selected, respectively, from among 2,4-dichloronitrobenzene and its derivatives which are chlorinated in position 3 or 5 or in positions 3 and 5, with an alkali metal fluoride, particularly potassium fluoride, in a polar aprotic solvent.

16. Process according to claim 15, characterised in that the compound or mixture of compounds of formula (IV) is obtained by reacting a compound or mixture of compounds of formula (V) 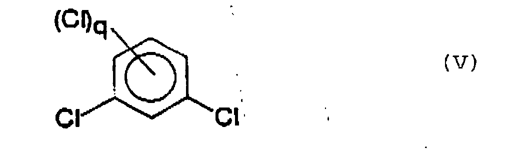 which are selected, respectively, from among 1,3-dichlorobenzene and its derivatives which are chlorinated in position 2 or 4 or in positions 2 and 4, with a nitration reagent for aromatic compounds, notably with nitric acid.

17. Process according to one of claims 1 to 13, for preparing 3,5-difluoroaniline of formula (Ib) 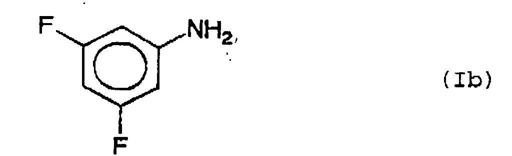 characterised in that a compound or mixture of compounds of formula (IIb) 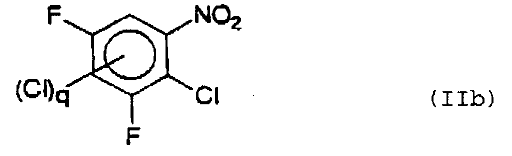 wherein q equals 0 to 2, these compounds being selected from among 2-chloro-3,5-difluoronitrobenzene and the derivatives thereof chlorinated in the 4 or 6 position or in the 4 and 6 positions, is or are subjected to catalytic hydrogenation.

18. Process according to claim 17, characterised in that the compound or mixture of compounds of formula (IIb) is obtained by reacting a compound or mixture of compounds of formula (VI) 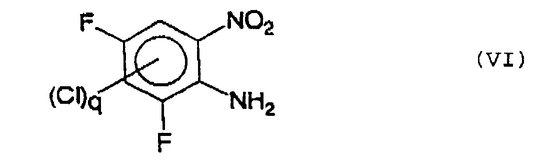 which are selected respectively from among 2-amino-3,5-difluoronitrobenzene and the derivatives thereof which are chlorinated in position 4 or 6 or in positions 4 and 6, with copper(I)chloride in the presence of nitrous acid.

19. Process according to claim 18, characterised in that the compound or mixture of compounds of formula (VI) is obtained by reacting a compound or mixture of compounds of formula (VII) 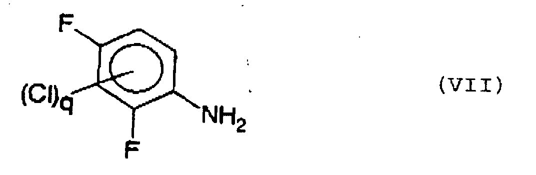 which are selected respectively from among 2,4-difluoroaniline and the derivatives thereof chlorinated in the 3 or 5 position or in the 3 and 5 positions, with a reagent for nitration of aromatic compounds, notably nitric acid.

20. Process according to claim 17, characterised in that the compound or mixture of compounds of formula (IIb) is obtained by reacting a compound or mixture of compounds of formula (VIII) 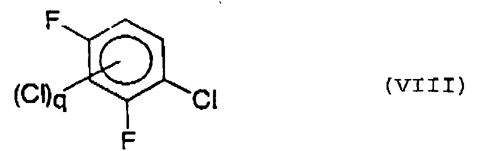 which are selected respectively from among 1-chloro-2,4-difluorobenzene and the derivatives thereof chlorinated in position 3 or 5 or in positions 3 and 5, with a nitration reagent for aromatic compounds, notably nitric acid.

21. Process according to claim 20, characterised in that the compound or mixture of compounds of formula (VIII) is obtained by reacting a compound or mixture of compounds of formula (VII) 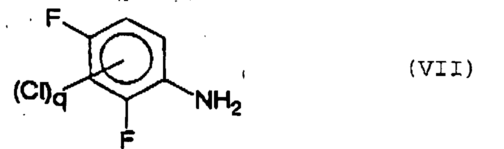 selected respectively from among 2,4-difluoroaniline and the derivatives thereof which are chlorinated in the 3 or 5 position or in the 3 and 5 positions with copper(I)chloride in the presence of nitrous acid.

22. Process according to claim 19 or 21, characterised in that the compound or mixture of compounds of formula (VII) is obtained by hydrogenation of a compound or mixture of compounds of formula (IIa) 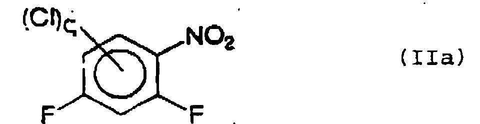 wherein q equals 0 to 2, which are selected from among 2,4-difluoronitrobenzene and the derivatives thereof chlorinated in the 3 or 5 position or in the 3 and 5 positions, notably using a process according to any one of claims 1 to 14.
